(19) Europäisches Patentamt
European Patent Office
Office européen des brevets

(11) Veröffentlichungsnummer: 0 172 141
B1

# (12) EUROPÄISCHE PATENTSCHRIFT

(45) Veröffentlichungstag der Patentschrift: 07.12.88

(51) Int. Cl.⁴: C 07 D 237/04, A 61 K 31/535

(21) Anmeldenummer: 85810369.0

(22) Anmeldetag: 12.08.85

## (54) Substituierte Pyridazinone, Verfahren zu ihrer Herstellung, pharmazeutische Präparate enthaltend diese Verbindungen und deren Verwendung.

(30) Priorität: 17.08.84 CH 3945/84

(43) Veröffentlichungstag der Anmeldung: 19.02.86 Patentblatt 86/8

(45) Bekanntmachung des Hinweises auf die Patenterteilung: 07.12.88 Patentblatt 88/49

(84) Benannte Vertragsstaaten: AT BE CH DE FR GB IT LI LU NL SE

(56) Entgegenhaltungen:
EP-A- 0 129 791
DE-A- 2 150 436
DE-A- 2 207 517

(73) Patentinhaber: CIBA-GEIGY AG, Klybeckstrasse 141, CH-4002 Basel (CH)

(72) Erfinder: Göschke, Richard, Dr., Felixhäglistrasse 21, CH-4103 Bottmingen (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue substituierte Pyridazinone, insbesondere 5-Hydroxymethyl-4,5-dihydro-3(2H)-pyridazinone, deren Herstellung, sowie pharmazeutische Präparate, die diese neuen Verbindungen enthalten, und auch ihre Verwendung.

In der DE-A-2 207 517 werden unter anderem im Phenylrest in p-Stellung durch einen Heterocyclus substituierte 6-Phenyl-4,5-dihydro-3(2H)-pyridazinone und in der DE-A-2 150 436 6-Phenyl-4,5-dihydro-3(2H)-pyridazinone oder die in 4- und/oder 5-Stellung durch eine oder zwei Methylgruppen substituierte Analoge beschrieben, die im Phenylrest durch einen Substituenten aus der Gruppe Cyan, Carboxamid, p-$NH_2$-, m-Nitro-, m-Amino- oder m-niedere Alkanoylamino substituiert sind, welche eine blutdrucksenkende Wirkung aufweisen.

Überraschenderweise wurde nun gefunden, dass die neuen Verbindungen der allgemeinen Formel I

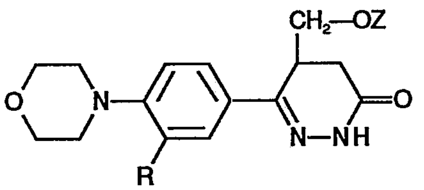

(I)

worin R ein Halogenatom, eine Niederalkyl-, Niederalkoxy-, die Nitro-, Hydroxy-, Cyano-, Carboxy-, Niederalkoxycarbonyl-, Carbamoyl- oder die Trifluormethylgruppe bedeutet, und Z Wasserstoff oder eine substituierte oder unsubstituierte Niederalkanoyl- oder eine Benzoyl- oder eine ein- oder mehrfach durch Halogen, Niederalkyl, Niederalkoxy, Hydroxy, Nitro oder Trifluormethyl substituierte Benzoylgruppe bedeutet, ihre Salze und ihre tautomeren Formen eine ausgeprägte antithrombotische Wirkung aufweisen.

Der Ausdruck «nieder» definiert in den oben oder nachfolgend genannten organischen Resten oder Verbindungen solche mit höchstens 7, vorzugsweise 4, insbesondere 1 oder 2 Kohlenstoffatomen.

R als Niederalkylrest ist z.B. Äthyl, Propyl, Isopropyl, Butyl , Isobutyl oder tert. Butyl und insbesondere Methyl; als Niederalkoxy, z.B. Äthoxy, Propoxy, Isopropoxy, Butoxy, Isobutoxy und insbesondere Methoxy; und als Halogen Brom, Jod, insbesondere Fluor oder vor allem Chlor.

R als Niederalkoxycarbonyl ist z.B. die Methoxycarbonyl- oder Äthoxycarbonylgruppe.

Eine unsubstituierte oder substituierte Niederalkanoylgruppe ist z.B. Acetyl, Propionyl oder auch Butyryl, Halogenniederalkanoyl wie z.B. Halogenacetyl, z.B. Chloracetyl. Der Phenylrest in der Benzoylgruppe kann gegebenenfalls ein- oder auch mehrfach durch Halogen, Niederalkyl, Niederalkoxy, Hydroxy, Nitro, Carboxy oder auch durch Trifluormethyl substituiert sein.

Die Verbindungen der Formel I weisen wertvolle pharmakologische Eigenschaften auf. So besitzen sie beispielsweise ausgeprägte antithrombotische Wirksamkeit. Diese kann beispielsweise am Meerschweinchen anhand der Hemmung der Thrombocytopenie nach Induktion durch ADP (Thromboembolism, Edited by J.R. Mitchell & J.G. Doment, Academic Press, (1977), p. 36) im Dosisbereich von etwa 30 bis 300 mg/kg p.o., anhand der Forssman-Reaktion (Thrombosis, Haemostasis 42, 100 (1979)) im Dosisbereich von etwa 100 bis 300 mg/kg p.o., und anhand der Hemmung der an einem Baumwollfaden sich bildenden Thrombose in einem extracorporealen shunt an der Ratte (Methode analog Brit. J. Pharmacol., 73, 219 P (1981)) im Dosisbereich von etwa 5 bis 50 mg/kg p.o., sowie anhand der Hemmung der ex vivo durch Collagen oder Arachindonsäure induzierten Plättchenaggregation nach vorausgegangener peroraler Applikation des Wirkstoffes in Dosen von 5 bis 100 mg/kg nachgewiesen werden. Die Verbindung der allgemeinen Formel I sind dementsprechend vorzüglich geeignet zur Behandlung thrombotischer Erkrankungen und können als Wirkstoffe in antithrombotischen Arzneimitteln verwendet werden.

Die Erfindung betrifft in erster Linie Verbindungen, in denen R ein Halogenatom, die Cyano-, Hydroxy-, Carboxy-, Niederalkyl-, Niederalkoxy- oder auch die Trifluormethylgruppe bedeutet und Z die Bedeutung von Wasserstoff oder auch Niederalkanoyl hat, ihre Salze und ihre tautomeren Formen.

Von besonderem Interesse in dieser Erfindung sind Verbindungen, in denen R ein Halogenatom, insbesondere Chlor, eine Niederalkylgruppe, insbesondere Methyl oder auch die Cyanogruppe bedeutet und Z die Bedeutung von Wasserstoff oder auch Niederalkanoyl hat, insbesondere Acetyl, und ihre tautomeren Formen.

Besonders zu nennen sind die in den Beispielen beschriebenen Verbindungen der Formel I und ihre tautomeren Formen.

Die neuen Pyridazinone der Formel I werden nach an sich bekannten Methoden hergestellt.

So kann man die neuen Verbindungen der Formel I erhalten, indem man eine Ketocarbonsäure der Formel II

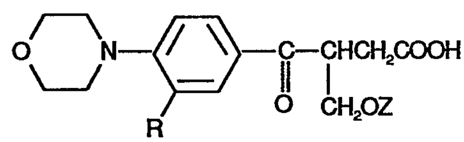

(II)

oder ein reaktionsfähiges Derivat einer solchen Ketocarbonsäure, worin R und Z die oben angegebene Bedeutung haben, mit Hydrazin umsetzt. Vorzugsweise verwendet man das in Hydratform vorliegende Hydrazinhydrat, welches im Überschuss verwendet auch gleichzeitig als Lösungsmittel dienen kann. Zweckmässiger ist es jedoch, ein zusätzliches inertes Lösungsmittel zuzusetzen. Als inerte Lösungsmittel eignen sich vorzugsweise Alkohole wie z.B. Methanol, Äthanol, Isopropanol, n-Butanol, Isoamylalkohol, Glykole und deren Äther wie Äthylenglykol, Diäthylenglykol, Äthylenglykolmonomethyl- oder -monoäthyläther (Methylglykol oder Äthylglykol), ferner Äther, insbesondere wasserlösliche Äther wie Tetrahydrofuran, Dioxan oder Äthylenglykoldimethyläther (Diglyme); ferner Wasser, sowie Gemische dieser Lösungsmittel untereinander, insbesondere Gemische mit Wasser, z.B. wässriges Äthanol. Die Reaktionstemperaturen liegen zweckmässig zwischen etwa 20 und etwa 200°C, vorzugsweise zwischen 60 und 80°C.

Als reaktionsfähige Derivate der Ketocarbonsäure der Formel II eignen sich beispielsweise die Ester, insbesondere Niederalkylester, wie z.B. Methyl- oder auch Äthylester. Ferner können noch die Säureamide und Säurehalogenide der Säuren der Formel II, insbesondere die Säurechloride oder Säurebromide verwendet werden. Vor allem eignen sich Lactone der Formel IIa

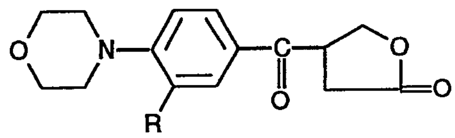

(IIa)

zum Umsatz mit Hydrazin oder einem Hydrazinhydrat.

Weitere geeignete reaktionsfähige Derivate der Carbonsäure der Formel II können während der Reaktion in situ gebildet werden. Dazu gehören beispielsweise die Hydrazone der Formel $R_1-C(=N-NH_2)-CH-(CH_2-OZ)-CH_2-COOH$, die Hydrazide der Formel $R_1-CO-CH(-CH_2-OZ)-CH_2-CO-NH-NH_2$ und die Hydrazone der Hydrazide der Formel

$$R_1—C(=N—NH_2)—CH(—CH_2—OZ)—CH_2—CO—NH—NH_2$$

worin $R_1$ die Bedeutung des Restes

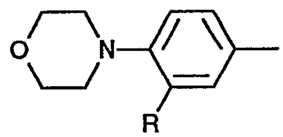

hat.

Die in situ gebildeten Ausgangsstoffe werden aus den Ketocarbonsäuren der Formel II hergestellt und werden aus dem Reaktionsgemisch nicht isoliert, sondern werden weiter zu den Verbindungen der Formel I umgesetzt.

Die Verbindungen der Formel I können auch erhalten werden, indem man Verbindungen der Formel III,

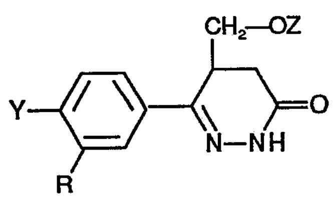

(III)

worin R und Z die unter der Formel I angegebenen Bedeutungen haben, und Y eine zusammen mit Wasserstoff abspaltbare Gruppe bedeutet, mit Morpholin umsetzt. Das verwendete Morpholin wird vorteilhafterweise als freie Base im Überschuss eingesetzt, kann jedoch auch in Form eines Säureadditionssalzes, beispielsweise als Hydrohalogenid, wie z.B. Hydrochlorid, verwendet werden.

Bei Verwendung eines nur geringen Überschusses des Morpholins als freie Base oder bei Verwendung des Morpholins als Säureadditionssalz ist es zweckmässig, eine stöchiometrisch äquivalente Menge z.B. eines tertiären Alkylamins, wie Triäthylamin oder N-Äthyldiisopropylamin, zusätzlich hinzusetzen.

Die beschriebene Umsetzung von Verbindungen der Formel III mit Morpholin erfolgt gegebenenfalls in Gegenwart eines Lösungsmittels, vorzugsweise eines aprotischen Lösungsmittels. Beispiele für bevorzugt verwendbare Lösungsmittel sind Äther, wie z.B. Diäthyläther und Tetrahydrofuran, insbesondere aliphatische Ketone und Ester, wie z.B. Aceton, Methyläthylketon und Äthylacetat, aromatische Kohlenwasserstoffe, wie z.B. Benzol, Toluol oder Xylol, sowie Acetonitril. Besonders bevorzugt kann die Umsetzung in Diäthyläther oder Acetonitril ausgeführt werden.

Die Umsetzungen können bei einer Temperatur zwischen 0–150°C, vorzugsweise jedoch zwischen Raumtemperatur und Rückflusstemperatur des Reaktionsgemisches ausgeführt werden.

Eine zusammen mit Wasserstoff abspaltbare Gruppe Y ist z.B. in erster Linie eine freie oder vorzugsweise verätherte Mercaptogruppe, ferner eine gegebenenfalls reaktionsfähige, funktionell abgewandelte Hydroxygruppe oder die Nitroaminogruppe. Eine verätherte Mercaptogruppe ist in erster Linie eine durch einen gegebenenfalls substituierten Kohlenwasserstoffrest, insbesondere aliphatischen Charakters, verätherte Mercaptogruppe. Sie stellt in erster Linie Niederalkylthio, z.B. Methylthio, Äthylthio oder Butylthio, ferner Phenylthio oder Phenylniederalkylthio, z.B. Benzylthio, dar. Eine gegebenenfalls reaktionsfähige, funktionelle abgewandelte Hydroxygruppe ist eine freie und u.a. eine entsprechende veresterte Hydroxygruppe. Eine solche ist u.a. Halogen, z.B. Chlor oder Brom, oder Niederalkylsulfonyloxy, z.B. Methansulfonyloxy.

Vorzugsweise bedeutet Y als eine zusammen mit Wasserstoff abspaltbare Gruppe ein Halogenatom, wie z.B. Chlor oder Brom.

Verbindungen der allgemeinen Formel I können auch hergestellt werden, indem man eine Verbindung der Formel IV

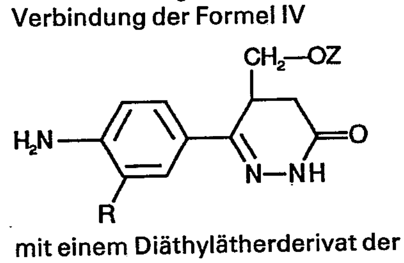

(IV)

mit einem Diäthylätherderivat der Formel V

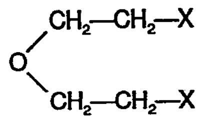

(V)

worin X eine zusammen mit Wasserstoff abspaltbare Gruppe bedeutet und R und Z die oben angegebenen Bedeutungen haben, umsetzt.

Die Umsetzung einer Verbindung der Formel IV mit einer Verbindung der Formel V erfolgt vorzugsweise in Gegenwart einer organischen Base wie z.B. eines tertiären Alkylamins, wie Triäthylamin oder N-Äthyldiisopropylamin.

Die beschriebene Umsetzung von Verbindungen der Formel IV mit einer Verbindung der Formel V erfolgt gegebenenfalls in Gegenwart eines Lösungsmittels, vorzugsweise eines polaren Lösungsmittels, wie z.B. Dimethylformamid.

Die Umsetzungen können bei einer Temperatur zwischen 0-200°C, vorzugsweise jedoch zwischen Raumtemperatur und Rückflusstemperatur des Reaktionsgemisches ausgeführt werden. Eine zusammen mit Wasserstoff abspaltbare Gruppe X ist weiter oben unter Formel III für das Symbol Y bereits definiert worden.

Vorzugsweise bedeutet X als eine zusammen mit Wasserstoff abspaltbare Gruppe ein Halogenatom, beispielsweise Chlor oder Brom.

Nach einem weiteren Verfahren können Verbindungen der Formel I, worin R ein Halogenatom oder die Cyanogruppe bedeutet, hergestellt werden, indem man eine Verbindung der Formel VI,

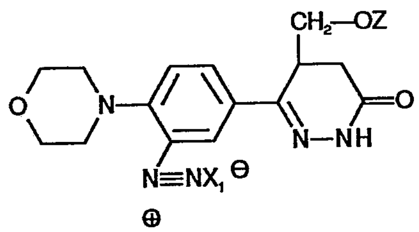

(VI)

worin $X_1^{\ominus}$ ein Anion einer Mineralsäure bedeutet, beispielsweise in Gegenwart von Kupfer oder eines Kupfer-I-salzes, beispielsweise eines Halogenids oder Cyanids erhitzt. Ein Anion einer Mineralsäure ist beispielsweise das Anion einer Halogenwasserstoffsäure. Im Falle der Einführung von R = Fluor ist $X_1^{\ominus}$ ein Fluorid- oder Tetrafluorboratanion. Die Erhitzung einer Verbindung der Formel VI erfolgt für den Fall, dass $X_1$ ein Fluorid- oder Tetrafluorboratanion ist, in Flusssäure oder in Tetrafluorborsäure. bei der Einführung einer Cyanogruppe setzt man beispielsweise das Diazoniumsalz der Formel VI mit Kupfer-I-cyanid, das in Kaliumcyanid komplex gelöst ist, um. Beispielsweise setzt man ein Diazoniumsalz der Formel VI mit einem Gemenge aus Kaliumcyanid und Kupfer-I-sulfat um. Die Freisetzung des Diazoniumsalzes erfolgt thermisch bei Temperaturen zwischen 30–150°C, vorzugsweise zwischen 30–40° bei Vorliegen eines Diazoniumfluorids und zwischen 100–150°C bei Vorliegen eines Diazoniumtetrafluorborates.

Die Diazotierung aromatischer Amine erfolgt beispielsweise mit einem Alkalimetall-, wie z.B. Natriumnitrit, vorzugsweise mit Hilfe von trockenem Natriumnitrit. Die Diazotierung wird beispielsweise bei einer Temperatur zwischen −10° und +10°, vorzugsweise bei einer Temperatur zwischen 0-5°C ausgeführt. Durch Umsetzung mit einer Mineralsäure erhält man Verbindungen der Formel VI, in der $X_1$ das Anion einer Mineralsäure bedeutet.

Nach einem weiteren Verfahren können Verbindungen der Formel I, worin R ein Halogenatom bedeutet, hergestellt werden, indem man Verbindungen der Formel VII

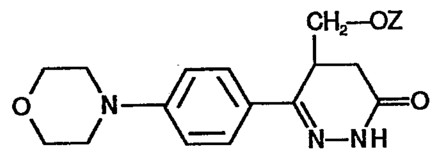

(VII)

halogeniert.

Die Halogenierung kann einerseits unter Verwendung von Halogen, vorzugsweise in Gegenwart einer Lewissäure, z.B. eines Eisen-III-, Aluminium-, Antimon-III- oder Zinn-IV-halogenids ausgeführt werden. Die Halogenierung kann andererseits auch mit Hilfe eines Halogenüberträgers, wie z.B. in Gegenwart eines Schwermetalls, beispielsweise Eisen oder unter Verwendung eines Halogenierungsmittels, wie z.B. Chlorwasserstoff in Gegenwart eines Oxydationsmittels, wie z.B. Wasserstoffperoxyd, oder eines Alkalimetall-, z.B. Natriumchlorat, eines Nitrosylhalogenids, z.B. Nitrosylchlorid oder -bromid oder eines N-Halogen-imids, z.B. Bromsuccinimid oder -phthalimid, ausgeführt werden.

Die direkte Einführung eines Jodatoms erfolgt bei Verwendung von Jodwasserstoff in Gegenwart eines Oxydationsmittels, beispielsweise in Gegenwart von Salpetersäure oder Quecksilberoxyd.

Die erwähnten Halogenierungen werden je nach Bedeutung des Halogenatoms bei Temperaturen zwischen −10°C und Rückflusstemperatur des Reaktionsgemisches, vorzugsweise bei Temperaturen zwischen -5° und 30°C ausgeführt.

Verbindungen der Formel I, in der R die unter Formel I angegebenen Bedeutungen hat und Z Wasserstoff bedeutet, können auch erhalten werden, indem man eine Verbindung der Formel VIII,

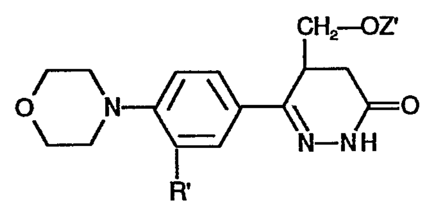

(VIII)

in der R′ die Bedeutung von R hat oder eine leicht solvolysierbare oder hydrogenolysierbare Äther- oder Acyloxygruppe bedeutet und -OZ′ eine leicht solvolysierbare oder hydrogenolysierbare

Äther- oder Acyloxygruppe bedeutet, solvolysiert oder hydrogenolysiert.

Eine leicht solvolysierbare oder auch hydrogenolysierbare Äther oder Acyloxygruppe ist z.B. eine durch Solvolyse, einschliesslich Hydrolyse, Acidolyse oder Alkoholyse oder mittels Reduktion, einschliesslich Hydrogenolyse abspaltbare Äther- oder auch Acyloxygruppe.

Eine durch Solvolyse abspaltbare Acyloxygruppe R′ oder auch -OZ′, ist beispielsweise eine Acyloxygruppe, in der der Acylteil der Rest einer organischen Carbonsäure, z.B. Niederalkanoyl, wie Acetyl, Halogenniederalkanoyl, wie Halogenacetyl, z.B. Chloracetyl, oder Carbamoyl, oder Aroyl, wie Benzoyl, ferner der Acylrest den Rest eines Halbesters der Kohlensäure, wie Niederalkoxycarbonyl, z.B. Methoxycarbonyl, Äthoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenyl-niederalkoxycarbonyl, z.B. Benzyloxycarbonyl oder Diphenylmethoxycarbonyl oder Aroylmethoxycarbonyl, z.B. Phenacyloxycarbonyl, ferner eine gegebenenfalls substituierte 1-Polyphenyl-niederalkylgruppe, worin Substituenten des Phenylteils, z.B. Niederalkyl oder Niederalkoxy, z.B. Methyl oder Methoxy sein können, und in erster Linie Trityl oder auch einen Organosilylrest, insbesondere Trimethylsilyl bedeuten.

Eine solvolytisch abspaltbare Äthergruppe ist z.B. Niederalkoxy, beispielsweise Methoxy oder Äthoxy, oder eine 1-Phenylniederalkoxygruppe, wie z.B. Benzyloxy. Diese Reste können durch Niederalkoxy, z.B. Methoxy oder Äthoxy, oder auch Niederalkoxyäthoxy, z.B. Methoxyäthoxy substituiert sein.

Benzyloxyreste als abspaltbare Äthergruppen können gegebenenfalls durch ein oder auch mehrere Substituenten, wie z.B. Niederalkyl, beispielsweise Methyl, Äthyl, Isopropyl oder n-Propyl, Halogen, beispielsweise Chlor oder Brom, oder auch Niederalkoxy, wie z.B. Methoxy oder Äthoxy, substituiert sein. Diese Substituenten sitzen vorzugsweise in ortho- oder auch in para-Stellung.

Ebenfalls solvolytisch, insbesondere hydrolytisch oder alkoholytisch in saurem Medium abspaltbar sind ihrerseits in α-Stellung durch eine Äthergruppe substituierte aliphatische Äthergruppen, wie Äthoxymethoxy, Butoxymethoxy oder 1-Äthoxyäthoxy, und insbesondere analoge cyclische Reste, z.B. 1-Oxacycloalkan-2-yloxygruppen, vor allem Tetrahydropyran-2-yloxy, ferner z.B. auch 4-Methoxytetrahydropyran-4-yloxy.

Wird die Solvolyse der Äther- oder auch Acyloxygruppen R′ und/oder -OZ′ hydrolytisch ausgeführt, so wird diese je nach Art der abspaltbaren Gruppen in Gegenwart einer organischen Säure, wie p-Toluolsulfonsäure oder einer Mineralsäure, wie Chlorwasserstoff- oder Schwefelsäure, oder eines Alkalimetall- oder Erdalkalimetallhydroxyds oder -carbonats, oder in Gegenwart von Ammoniak oder eines Amins wie Isopropylamin, oder Hydrazinhydrat ausgeführt. Wird die Solvolyse mittels einer der obengenannten Säuren in einem Alkohol, beispielsweise mittels p-Toluolsulfonsäure in Äthylalkohol ausgeführt, so erfolgt die Solvolyse alkoholytisch.

Ähergrupppen, wie z.B. Niederalkoxygruppen, insbesondere Methoxy oder Äthoxy, können in Lösung oder Schmelze mittels eines Metallhalogenids wie z.B. Aluminium- oder auch Borhalogenids, beispielsweise Aluminiumtrichlorid, Aluminiumtribromid, Bortrichlorid, oder auch Bortribromid, abgespalten werden. Als Lösungsmittel eignen sich beispielsweise Benzol, Nitrobenzol oder auch Äthylenchlorid. (Vgl. Jour. Chem. Soc. (1961), 1008; Ber. (1943), 76B, 900; Jour. Org. Chem. (1962), 27, 2037; Ber. (1960), 92, 2761; Jour. Am. Chem. Soc. (1968), 24, 2289; Tetr. Lett. (1966), 4155).

Acidolytisch abspaltbar sind Acyloxygruppen, in denen der Acylteil ein Acylrest von Halbester der Kohlensäure, wie z.B. tert.-Niederalkoxycarbonyl, oder gegebenenfalls substituiertes Diphenylmethoxycarbonyl bedeutet. Ferner können auch Äthergruppen wie z.B. tert.-Niederalkoxygruppen acidolytisch abgespalten werden. Die acidolytische Abspaltung kann durch Behandeln mit geeigneten starken organischen Carbonsäuren, wie gegebenenfalls durch Halogen, insbesondere Fluor, substituierten Niederalkansäuren, in erster Linie Trifluoressigsäure (wenn notwendig in Gegenwart eines aktivierenden Mittels, wie Anisol), sowie mit Ameisensäure erfolgen. Die obigen Reaktionen werden, wenn nicht schon vorher erwähnt, in Gegenwart eines Lösungsmittels oder Lösungsmittelgemisches durchgeführt, wobei geeignete Reaktionsteilnehmer auch als solche dienen können.

Eine durch Reduktion, insbesondere durch Hydrogenolyse abspaltbare Äthergruppe ist in erster Linie eine α-Arylniederalkylgruppe, wie eine gegebenenfalls substituierte 1-Phenylniederalkylgruppe, worin Niederalkyl bis zu 7 Kohlenstoffatome hat und worin Substituenten, insbesondere des Phenylteils, z.B. Niederalkyl oder Niederalkoxy mit jeweils bis zu 7 Kohlenstoffatomen, z.B. Methyl oder Methoxy, sein können, und ganz besonders jedoch Benzyl.

Die reduktive Abspaltung der Äthergruppen kann insbesondere z.B. durch Behandeln mit katalytisch aktiviertem Wasserstoff, wie Wasserstoff in Gegenwart eines geeigneten Hydrierkatalysators, z.B. Nickel-, Platin- oder Palladium-, aber auch eines Rhodium- oder Rutheniumkatalysators erfolgen, oder man arbeitet mit einem Hydrid reduktionsmittel, wie z.B. Lithiumaluminiumhydrid.

Unter hydrogenolytisch abspaltbaren Acyloxyresten werden solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyloxy, wie 2,2,2-Trichloräthoxycarbonyloxy, die z.B. mit einem reduzierenden Schwermetall, wie z.B. Zink, oder mit einem reduzierenden Schwerme-

tallsalz, wie Chrom(II)-salz, z.B. -chlorid oder -azetat, üblicherweise in Gegenwart einer organischen Carbonsäure, wie Ameisensäure oder Essigsäure, abgespalten werden.

Die obigen Reduktionsreaktionen werden in an sich bekannter Weise, üblicherweise in Gegenwart eines inerten Lösungsmittels, und, wenn notwendig unter Kühlen oder Erwärmen, z.B. in einem Temperaturbereich von etwa -20° bis etwa 150°C, und/oder in einem geschlossenen Gefäss unter Druck durchgeführt.

Je nach vorhandener Äther- oder auch Acyloxygruppe wird vorzugsweise die schonendste der beschriebenen Solvolyse- oder auch Hydrogenolysemethoden gewählt, um Veränderungen am Pyridazinongerüst zu vermeiden.

In erhaltenen Verbindungen der Formel I kann man im Rahmen der Definition der Endprodukte Substituenten einführen, abwandeln oder abspalten.

Beispielsweise lassen sich erhaltende Verbindungen der Formel I, in der R eine Hydroxygruppe bedeutet, in an sich bekannter Weise durch Umesterung oder Verätherung in Verbindungen der Formel I umwandeln, in der R ein Halogenatom oder eine Methoxygruppe bedeutet.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der Atmosphäre eines inerten Gases, wie z.B. Stickstoff, erfolgen.

Die Ausgangsstoffe sind bekannt, oder falls sie neu sind, lassen sich nach an sich bekannten Methoden herstellen. Wo es sich als zweckdienlich erweist, sind die verwendeten Ausgangsprodukte im Anschluss an das beschriebene Verfahren bereits beschrieben worden.

Ketocarbonsäuren der Formel II können nach an sich bekannten Methoden hergestellt werden, beispielsweise für Verbindungen der Formel II, in denen R ein Halogenatom oder auch eine Cyanogruppe bedeutet, durch Halogenierung oder Einführung einer Cyanogruppe in einer Verbindung der Formel X

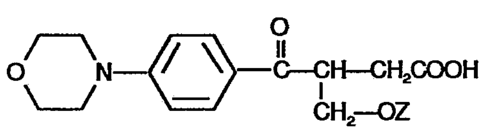 (X)

nach den oben beschriebenen Methoden, die in analoger Weise ausgeführt werden.

Die als Derivate verwendeten Lactone der Formel IIa lassen sich aus der entsprechend durch den Substituenten R substituierten 3-(4-Morpholino-benzoyl)-propionsäure durch Umsatz mit Formaldehyd gemäss «Synthesis», Georg Thieme Verlag, (Oktober 1980), S. 825–827 erhalten.

Verbindungen der allgemeinen Formel III sind bekannt oder können nach der von J.D. Albright et al, J. Het. Chem. 15, 881 (1978) beschriebenen Methode aus der entsprechenden Ketocarbonsäure durch Umsetzung mit Hydrazin erhalten werden. In analoger Weise können auch die Ausgangsverbindungen der allgemeinen Formel IV aus den entsprechenden Aminoketocarbonsäuren erhalten werden. Auch die Ausgangsverbindungen der allgemeinen Formel V sind bekannt und können beispielsweise aus den entsprechenden Dihydroxydiäthyläthern durch Veresterung mit einer Säure, beispielsweise Halogenwasserstoffsäure, erhalten werden. Diäthylätherderivate der Formel V lassen sich auch aus entsprechend substituierten Alkoholen der Formel $OH-CH_2-CH_2-X$ durch Verätherung erhalten.

Die Ausgangsverbindung der Formel VI lässt sich aus der entsprechenden Nitroverbindung durch Reduktion in die Aminoverbindung und nachfolgende Diazotierung herstellen.

Verbindungen der allgemeinen Formel VIII und IX können nach der im ersten Verfahren beschriebenen Methode aus den entsprechenden Ketocarbonsäuren durch Umsetzung mit Hydrazin erhalten werden.

Die Erfindung betrifft auch diejenigen Ausführungsformen eines Verfahrens, bei denen man ein Verfahren auf irgendeiner Stufe abbricht oder bei denen man von einer auf irgendeiner Stufe als Zwischenprodukt erhältlichen Verbindung ausgeht und die fehlenden Schritte durchführt, oder einen Ausgangsstoff unter den Reaktionsbedingungen bildet oder gegebenenfalls in Form eines Salzes verwendet. Die Erfindung beinhaltet auch daraus resultierende neue Zwischenprodukte.

Von der Erfindung ebenfalls umfasst sind therapeutische Stoffzusammenstellungen, die aus einem antithrombotisch wirksamen Anteil der Verbindungen der Formel I und einem pharmakologisch annehmbaren festen Trägerstoff oder flüssigen Verdünnungsmittel bestehen.

Eine erhaltende Verbindung der Formel I, in der R eine Carboxygruppe bedeutet, kann in an sich bekannter Weise, z.B. durch Umsetzen mit einer etwa stöchiometrischen Menge eines geeigneten salzbildenden Mittels, wie Ammoniak, einem Amin oder einem Alkalimetall- oder Erdalkalimetallhydroxyd, -carbonat oder hydrogencarbonat, in ein Salz umgewandelt werden. So erhältliche Ammonium- oder Metallsalze lassen sich durch Behandeln mit einer Säure, z.B. Salzsäure, Schwefelsäure oder auch Essigsäure, bis zum Erreichen des notwendigen pH-Wertes in die freie Säure überführen.

Die Säureadditionssalze der neuen Verbindungen können in an sich bekannter Weise in die freien Verbindungen übergeführt werden, z.B. mit basischen Mitteln, wie Alkalien oder Ionenaustauschern. Andererseits können die erhaltenen freien Basen mit organischen oder anorganischen Säuren Salze bilden.

Die erfindungsgemässen pharmazeutischen Präparate enthalten mindestens eine Verbindung der Formel I als Wirkstoff zusammen mit einem üblichen pharmazeutischen Trägerstoff. Die Art der Trägerstoffe richtet sich weitgehend nach

dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung von Thrombose kommen insbesondere feste Doseneinheitsformen, wie Tabletten, Dragées und Kapseln in Frage, die vorzugsweise zwischen 10 und 90% eines Wirkstoffes der allgemeinen Formel I enthalten, um die Verabreichung von täglichen Dosen zwischen 1,0 bis 1000 mg/kg, vorzugsweise zwischen 2 und 100 mg/kg, insbesondere zwischen 5 bis 10 mg/kg, an Warmblüter von etwa 70 kg Körpergewicht zu ermöglichen. Zur Herstellung von Tabletten und Dragéekernen kombiniert man die Verbindungen der Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschliessend beispielsweise mit konzentrierten Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Überzügen können Farbstoffe zugefügt werden, z.B. zur Kennzeichnung verschiedener Wirkstoffdosen. Weiche Gelatinekapseln und andere geschlossene Kapseln bestehen beispielsweise aus einem Gemisch von Gelatine und Glycerin und können z.B. Mischungen einer Verbindung der Formel I mit Polyäthylenglykol enthalten. Steckkapseln enthalten z.B. Granulate eines Wirkstoffes mit festen, pulverförmigen Trägerstoffen, wie z.B. Lactose, Saccharose, Sorbit, Mannit; Stärken, wie Kartoffelstärke, Maisstärke oder Amylopektin, Cellulosederivate sowie Magnesiumstearat oder Stearinsäure.

Als Doseneinheitsformen für die rektale Anwendung kommen z.B. Suppositorien in Betracht, welche aus einer Kombination eines Wirkstoffes mit einer Suppositorien-Grundmasse auf der Basis von natürlichen oder synthetischen Triglyceriden (z.B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Für Flüssigkeiten zur oralen Einnahme, wie Sirups und Elixiere, wird die Konzentration des Wirkstoffes derart gewählt, dass eine Einzeldosis leicht abgemessen werden kann, z.B. als Inhalt eines Teelöffels oder eines Messlöffels von z.B. 5 ml, oder auch als Mehrfaches dieser Volumina.

Die nachfolgenden Beispiele a) bis e) sollen die Herstellung einiger typischer Applikationsformen erläutern, jedoch keineswegs die einzigen Ausführungsformen von solchen darstellen.

a) 100,0 g Wirkstoff werden mit 610,0 g Lactose und 442,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und presst die Mischung zu 10'000 Tabletten von je 125 mg Gewicht und 10 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10'000 Dragéekernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1.5 g Farbstoff überzogen und getrocknet. Die erhaltenen Dragées wiegen je 150 mg und enthalten je 10 mg Wirkstoff.

c) 10,0 g Wirkstoff und 1990 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25% Wirkstoffgehalt löst man in 3 Litern dest. Wasser 1,5 Liter Glycerin, 42 g p-Hydroxybenzoesäuremethylester, 18 g p-Hydroxybenzoesäure-n-propylester und unter leichtem Erwärmen 25,0 g Wirkstoff, fügt 4 Liter 70%ige Sorbitlösung, 1000 g krist. Saccharose, 350 g Glucose und einen Aromastoff, z.B. 250 g «Orange Peel Soluble Fluid» von Eli Lilly und Co., Indianapolis, oder 5 g natürliches Zitronenaroma und 5 g «Halb und Halb»-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5% Wirkstoffgehalt löst man 150,0 g Wirkstoff und 30 g Natriumcyclamat in einem Gemisch von 4 Liter Äthanol (96%) und 1 Liter Propylenglykol. Andererseits mischt man 3,5 Liter 70%ige Sorbitlösung mit 1 Liter Wasser und fügt die Mischung zur obigen Wirkstofflösung. Hierauf wird ein Aromastoff, z.B. 5 g Hustenbonbon-Aroma oder 30 g Grapefruit-Essenz, beide von der Firma Haarmann und Reimer, Holzminden, Deutschland zugegeben, das Ganze gut gemischt, filtriert und mit dest. Wasser auf 10 Liter ergänzt.

Die nachfolgenden Beispiele erläutern die Herstellung der neuen Verbindungen der Formel I, sollen jedoch den Umfang der Erfindung in keiner Weise beschränken. Die Temperaturen sind in Celsiusgraden angegeben.

Beispiel 1

11,2 g 4-(3-Cyano-4-morpholino-benzoyl)-butyrolacton werden zusammen mit 2,55 ml Hydrazinhydrat und 170 ml Äthanol 16 Stunden am Rückfluss gehalten. Danach werden 29,7 ml Eisessig zugegeben und weitere 5 Stunden am

Rückfluss gerührt. Anschliessend wird das Reaktionsgemisch unter Rühren auf Raumtemperatur abgekühlt und die dabei ausgefällten Kristalle werden abgenutscht und mit wenig Äthanol und Diäthyläther nachgewaschen. Man erhält 5,6 g 6-(3-Cyano-4-morpholino-phenyl)-5-hydroxymethyl-4,5-dihydro-3(2H)-pyridazinon vom Smp. 217–218°.

Das als Ausgangsmaterial verwendete 4-(3-Cyano-4-morpholino-benzoyl)-butyrolacton wird wie folgt hergestellt: 10 g 3-(3-Cyano-4-morpholino-benzoyl)-propionsäure werden zusammen mit 2,98 g 35%iger Formaldehydlösung und 76,4 ml 0,5 N Natronlauge 16 Stunden bei Raumtemperatur gerührt. Danach wird das Reaktionsgemisch unter Rühren und Kühlen mit Eiswasser mit konzentrierter Salzsäure sauer gestellt und nochmals 16 Stunden bei Raumtemperatur gerührt. Es wird Äthylacetat zugegeben, bis sich zwei klare Phasen bilden. Die wässrige Phase wird abgetrennt und mit Äthylacetat extrahiert. Die organischen Phasen werden zweimal mit 2N Sodalösung, mit Wasser und mit Sole gewaschen, über Natriumsulfat getrocknet und eingedampft. Das als öligen Rückstand erhaltene 4-(3-Cyano-4-morpholino-benzoyl)-butyrolacton wird direkt in der Folgestufe eingesetzt.

Beispiel 2

Analog Beispiel 1 erhält man: Aus der 3-(3-Chlor-4-morpholino-benzoyl)-propionsäure das 6-(3-Chlor-4-morpholino-phenyl)-5-hydroxymethyl-4,5-dihydro-3(2H) pyridazinon vom Smp. 176–178°.

Aus der 3-(3-Nitro-4-morpholino-benzoyl)-propionsäure das 6-(3-Nitro-4-morpholino-phenyl)-5-hydroxymethyl-4,5-dihydro-3(2H) pyridazinon vom Smp. 216–217°.

Beispiel 3

1 g 6-(3-Cyano-4-morpholino-phenyl)-5-hydroxymethyl-4,5-dihydro-3(2H)-pyridazinon werden zusammen mit 0,3 ml Essigsäureanhydrid, 0,66 ml Triäthylamin, 20 ml Chloroform und 20 ml Dimethylformamid 25 Stunden bei 70° gerührt, wobei nach 5 und nach 20 Stunden jeweils noch 0,3 ml Essigsäureanhydrid zugegeben werden. Das Reaktionsgemisch wird zur Trockene eingedampft und der Rückstand wird auf Silicagel im System Äthylacetat:Methanol = 99:1 chromatographiert. Man erhält 1 g 6-(3-Cyano-4-morpholino-phenyl)-5-acetoxy-methyl-4,5-dihydro-3(2H)-pyridazinon als amorphen Festkörper (Schaum) mit einem Schmelzpunkt von 60–69° ($R_f$ im System Äthylacetat:Methanol = 9:1 ist 0,48).

Beispiel 4

45 g 3-Chlor-4-amino-benzoyl-propionsäure werden zusammen mit 50 ml 35%iger Formaldehydlösung und 440 ml 0,5 N Natronlauge 17 Stunden lang bei Raumtemperatur gerührt.

Das Reaktionsgemisch wird mit 2N Salzsäure auf pH 5 gestellt und mit Essigester extrahiert. Die Essigesterphasen werden mit Wasser gewaschen, über Natriumsulfat getrocknet und eingedampft. Der Rückstand, 3-(3-Chlor-amino-benzoyl)-3-hydroxymethyl-propionsäure wird direkt ungereinigt weiter umgesetzt, indem man diesen zusammen mit 1600 ml Äthanol und 18 ml Hydrazinhydrat 5 Stunden lang unter Rückfluss erhitzt. Das Reaktionsgemisch wird eingedampft und der Rückstand wird zwischen 4N Salzsäure und Essigester verteilt. Die wässrigen Phasen werden mit 2N Sodalösung basisch gestellt und mit Essigester extrahiert. Die Essigesterextrakte werden noch zweimal mit 2N Sodalösung und mit Wasser gewaschen, über Natriumsulfat getrocknet und eingedampft. Als Rückstand erhält man das rohe 6-(3-Chlor-4-amino-phenyl)-5-hydroxymethyl-4,5-dihydro-3(2H)-pyridazinon, welches zusammen mit 25 g β, β-Dibromdiäthyläther, und 37 ml Diisopropyläthylamin in 150 ml Dimethylformamid 18 Stunden lang unter Rückfluss erhitzt wird. Das Reaktionsgemisch wird am Rotationsverdampfer am Hochvakuum eingedampft. Der Rückstand wird in Essigester aufgenommen und mit 1N Salzsäure und Wasser mehrmals gewaschen. Die organische Phase wird über Natriumsulfat getrocknet und eingeengt. Dabei kristallisiert das 6-(3-Chlor-4-morpholino-phenyl)-5-hydroxymethyl-4,5-dihydro-3(2H)-pyridazinon aus. Der Smp. beträgt 172–173°.

Beispiel 5

26,3 g p-Morpholino-benzoyl-propionsäure werden zusammen mit 25 ml 35%iger Formaldehydlösung und 220 ml 0,5N Natronlauge 18 Stunden bei Raumtemperatur gerührt.

Das Reaktionsgemisch wird mit 2N Salzsäure auf pH 3 gestellt und mit Essigester extrahiert. Die Essigesterphasen werden mit Wasser gewaschen, über Natriumsulfat getrocknet und eingedampft. Der Rückstand, die rohe 3-(p-Morpholino-benzoyl), 3-Hydroxymethyl-propionsäure wird zusammen mit 9 ml Hydrazinhydrat und 800 ml Äthanol 5 Stunden zum Rückfluss erhitzt. Danach wird das Reaktionsgemisch am Rotationsverdampfer eingedampft. Der Rückstand wird in Essigester aufgenommen und die organischen Phasen werden mit 2N Soda und Wasser mehrmals gewaschen, dann über Natriumsulfat getrocknet und eingedampft. Der Rückstand wird auf Silicagel mit einem Gemisch aus Essigester: Methanol = 9:1 chromatographiert.

Das so erhaltene 6-(4-Morpholino-phenyl)-5-hydroxymethyl-4,5-dihydro-3-(2H)-pyridazinon wird in einem Gemisch aus 16 ml Eisessig und 20 ml konzentrierter Salzsäure gelöst und unter Rühren bei -10° mit der Lösung von 1,8 g Natriumchlorat in 2 ml Wasser versetzt. Das Gemisch wird 30 Minuten bei 0° und 30 Minuten bei Raumtemperatur weitergerührt und dann auf 400 ml Eiswasser gegossen. Die dabei entstehende Suspension wird mit konzentrierter NaOH auf pH 6 gestellt und mit Essigester extrahiert. Die Essigesterextrakte werden mit 1N Natriumbikarbonatlösung und mit Wasser gewaschen, über Natriumsulfat getrocknet und eingedampft. Der Rückstand wird auf Silicagel mit einem Gemisch aus Essigester:Methanol = 9:1 gereinigt. Durch Umkristallisieren der entsprechenden Fraktionen aus Essigester erhält man das 6-(-3Chlor-4-morpholino-phenyl)-5-hydroxy-methyl-4,5-dihydro-3(2H)-pyridazinon. Smp. 173-175°.

Beispiel 6

10 g 6-(3-Nitro-4-morpholino-phenyl)-5-hydroxymethyl-4,5-dihydro-3-(2H)-pyridazinon werden mit 1 g Katalysator Palladium auf Kohle in 200 ml Äthanol bei Raumtemperatur und Niederdruck hydriert.

Zur Aufarbeitung wird der Katalysator in 1N Salzsäure aufgeschlämmt, abgenutscht und mit 1N Salzsäure nachgewaschen. Das Filtrat wird mit 2N Natronlauge auf pH 6 gestellt und mit Essigester extrahiert. Die organischen Phasen werden mit Sole gewaschen, über Natriumsulfat getrocknet und eingedampft.

Der Rückstand, das rohe 6-(3-Amino-4-morpholino-phenyl)-5-hydroxy-methyl-4,5-dihydro-3(2H)-pyridazinon wird in einem Gemisch aus 90 ml konzentrierter Salzsäure und 90 ml Wasser gelöst und bei 0-5° unter Rühren mit der Lösung von 2,9 g Natriumnitrit in 10 ml Wasser diazotiert, wobei nach 15 Minuten mit Harnstoff das überschüssige Nitrit vernichtet wird. Nun wird die Lösung von 18 ml Kupfer(I)-chlorid in einem Gemisch aus 90 ml Wasser und 90 ml konzentrierter Salzsäure rasch zugegeben. Nach beendeter Zugabe wird das Reaktionsgemisch 3 Std. bei Raumtemperatur und weitere 2 Stunden bei 40° weitergerührt, anschliessend auf pH 10 gestellt und mit Essigester extrahiert. Die Essigesterphasen werden mit Wasser und Sole gewaschen, über Natriumsulfat getrocknet und eingedampft. Durch Reinigen des Rückstandes auf Silicagel mit einem Gemisch aus Essigester:Methanol = 9:1 und Umkristallisieren aus Essigester erhält man das 6-(3-Chlor-4-morpholino-phenyl)-5-hydroxymethyl-4,5-dihydro-3(2H)-pyridazinon, Smp. 174-176°.

Beispiel 7

25 g 3-Nitro-4-chlor-benzoyl-propionsäure werden zusammen mit 25 ml 35%iger Formaldehydlösung und 220 ml 0,5N Natronlauge 16 Stunden bei Raumtemperatur gerührt. Das Reaktionsgemisch wird mit 2N Salzsäure auf pH 3 gestellt und mit Essigester extrahiert. Die Essigesterphasen werden mit Wasser gewaschen, über Natriumsulfat getrocknet und eingedampft. Die als Rückstand erhaltene 3-(3-Nitro-4-chlor-benzoyl)-3-hydroxymethyl-propionsäure wird zusammen mit 9 ml Hydrazinhydrat und 800 ml Äthanol 5 Stunden am Rückfluss gekocht. Danach wird das Reaktionsgemisch eingedampft und der Rückstand wird zusammen mit 170 ml Dimethylsulfoxid, 10 ml Morpholin und 15 ml Diisopropyläthylamin während 3 Stunden bei 80° gerührt. Das Reaktionsgemisch wird dann am Rotationsverdampfer am Hochvakuum eingedampft und der Rückstand wird in 90 ml Methylenchlorid 30 Minuten zum Rückfluss erhitzt. Durch Abkühlen auf 0° kristallisiert unter Rühren das 6-(3-Nitro-4-morpholino-phenyl)-5-hydroxymethyl-4,5-dihydro-3(2H)-pyridazinon aus, Smp. 210-213°.

Beispiel 8

Zu einer Lösung von 1,66 ml Natriumhydriddispersion in 50 ml Dimethylformamid wird die Lösung von 5 g 3-(3-Cyano-4-morpholino-benzoyl)-propionsäure in 50 ml Dimethylformamid zugetropft. Das Reaktionsgemisch wird 30 Minuten bei 70° gerührt und dann bei -70° mit 7,2 ml Benzyl-chlormethyläther unter Rühren tropfenweise versetzt. Es wird 1 Stunde bei -70° und 16 Stunden bei Raumtemperatur weitergerührt. Anschliessend werden hintereinander 30 ml Wasser, 10 ml 2N Salzsäure und 50 ml Wasser zugegeben, das Gemisch mit Äther extrahiert und die Ätherphasen nach Waschen mit Wasser und Trocknen über Natriumsulfat eingedampft.

Der Rückstand wird mit 4 ml Hydrazinhydrat und 200 ml 50%ige Essigsäure zusammen 2 Stunden bei 100° und 16 Stunden bei Raumtemperatur gerührt. Das Gemisch wird am Rotationsverdampfer eingedampft und der Rückstand wird in Essigester aufgenommen. Die organischen Phasen werden mit Wasser, 1N Natriumbikarbonatlösung und Sole gewaschen, über Natriumsulfat getrocknet und eingedampft.

Aus dem Rückstand erhält man durch Chromatographie auf Silicagel mit einem Gemisch aus Toluol:Essigester = 1:1 das 6-(3-Cyano-4-morpholino-phenyl)-5-benzyloxymethyl-4,5-dihydro-3(2H)-pyridazinon vom Smp. 156-167°.

1 g 6-(3-Cyano-4-morpholino-phenyl)-5-benzyloxymethyl-4,5-dihydro-3(2H)-pyridazinon werden in 100 ml Äthanol mit 0,3 g Katalysator Palladium auf Kohle hydriert. Der Katalysator wird abgenutscht und das Filtrat eingedampft. Der Rückstand wird in 8 ml Methylenchlorid 30 Minuten zum Rückfluss erhitzt und auf 0° abgekühlt. Unter Rühren kristallisiert das 6-(3-Cyano-4-morpholino-phenyl)-5-hydroxy-methyl-4,5-dihydro-3(2H)-pyridazinon aus. nach dem Umkristallisieren aus Äthanol-Dimethylformamid beträgt der Smp. 219-221°.

Beispiel 9

86,5 g 3-(-3- Cyano-4-morpholino-benzoyl)-propionsäure werden zusammen mit 79 ml 35%iger Formaldehydlösung und 660 ml 0,5N Natronlauge 17 Stunden bei Raumtemperatur gerührt. Danach wird mit 160 ml 2N Salzsäure der pH-Wert auf 3 gestellt und das Gemisch wird mit Essigester extrahiert. Die organischen Phasen werden mit Natriumsulfat getrocknet und eingedampft. Der Rückstand wird zusammen mit 28 ml Hydrazinhydrat und 2850 ml Äthanol 5 Stunden am Rückfluss gerührt und dann am Rotationsverdampfer eingedampft. Der Rückstand wird in 900 ml Methylenchlorid suspendiert und 30 Minuten zum Rückfluss erhitzt. Nach dem Abkühlen auf 0° kristallisiert das Produkt aus. Man erhält das 6-(3-Cyano-4-morpholino-phenyl-)-5-hydroxymethyl-4,5-dihydro-3(2H)-pyridazinon vom Smp. 213-215°. Durch Umkristallisieren aus

400 ml Methylenchlorid mit 400 ml Äthanol steigt der Smp. auf 221–222°.

**Patentansprüche für die Vertragsstaaten: DE, GB, FR, CH, LI, IT, NL, BE, SE, LU**

1. Pyridazinone der allgemeinen Formel I

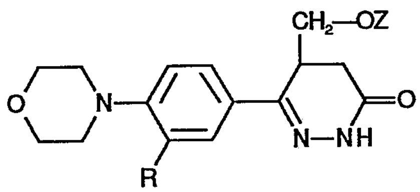

(I)

worin R ein Halogenatom, eine Niederalkyl-, Niederalkoxy-, die Nitro-, Hydroxy- Cyano-, Carboxy-, Niederalkoxycarbonyl-, Carbamoyl- oder die Trifluormethylgruppe bedeutet, und Z Wasserstoff oder eine substituierte oder unsubstituierte Niederalkanoyl- oder eine Benzoyl- oder eine ein- oder mehrfach durch Halogen, Niederalkyl, Niederalkoxy, Hydroxy, Nitro oder Trifluormethyl substituierte Benzoylgruppe bedeutet, ihre Salze und ihre tautomeren Formen, wobei die mit «nieder» bezeichneten Reste höchstens 7 Kohlenstoffatome aufweisen.

2. Pyridazinone der allgemeinen Formel I gemäss Anspruch 1, worin R ein Halogenatom, die Cyano-, Hydroxy-, Carboxy-, Niederalkyl-, Niederalkoxy- oder auch die Trifluormethylgruppe bedeutet und Z die Bedeutung von Wasserstoff oder auch Niederalkanoyl hat, ihre Salze und ihre tautomeren Formen, wobei die mit «nieder» bezeichneten Reste höchstens 7 Kohlenstoffatome aufweisen.

3. Pyridazinone der allgemeinen Formel I gemäss Anspruch 1, worin R ein Halogenatom, insbesondere Chlor, eine Niederalkylgruppe, insbesondere Methyl oder auch die Cyanogruppe bedeutet und Z die Bedeutung von Wasserstoff oder auch Niederalkanoyl hat, insbesondere Acetyl, und ihre tautomeren Formen, wobei die mit «nieder» bezeichneten Reste höchstens 7 Kohlenstoffatome aufweisen.

4. 6-(3-Cyano-4-morpholino-phenyl)-5-hydroxymethyl-4,5-dihydro-3(2H)-pyridazinon und seine tautomeren Formen.

5. 6-(3-Chlor-4-morpholino-phenyl)-5-hydroxymethyl-4,5-dihydro-3(2H)-pyridazinon und seine tautomeren Formen.

6. 6-(3-Nitro-4-morpholino-phenyl)-5-hydroxymethyl-4,5-dihydro-3(2H)-pyridazinon und seine tautomeren Formen.

7. 6-(3-Cyano-4-morpholino-phenyl)-5-acetoxymethyl-4,5-dihydro-3(2H)-pyridazinon und seine tautomeren Formen.

8. Pharmazeutische Präparate enthaltend eine der Verbindungen der allgemeinen Formel I gemäss Anspruch 1 und übliche Trägerstoffe.

9. Die Verbindungen der allgemeinen Formel I gemäss Anspruch 1 zur Anwendung in einem Verfahren zur therapeutischen Behandlung des menschlichen und tierischen Körpers.

10. Die Verbindungen der allgemeinen Formel I gemäss Anspruch 1 zur Verwendung als antithrombotisch wirksames Mittel.

11. Die Verwendung von Verbindungen der allgemeinen Formel I gemäss Anspruch 1 und übliche Trägerstoffe zur Herstellung von pharmazeutischen Präparaten.

12. Verfahren zur Herstellung von Verbindungen der allgemeinen Formel I

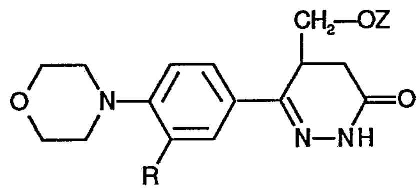

(I)

worin R ein Halogenatom, eine Niederalkyl-, Niederalkoxy-, die Nitro-, Hydroxy-, Cyano-, Carboxy-, Niederalkoxycarbonyl-, Carbamoyl- oder die Trifluormethylgruppe bedeutet, und Z Wasserstoff oder eine substituierte oder unsubstituierte Niederalkanoyl- oder Benzoyl- oder eine ein- oder mehrfach durch Halogen, Niederalkyl, Niederalkoxy, Hydroxy, Nitro oder Trifluormethyl substituierte Benzoylgruppe bedeutet, ihre Salze und ihre tautomeren Formen, wobei die mit «nieder» bezeichneten Reste höchstens 7 Kohlenstoffatome aufweisen, dadurch gekennzeichnet, dass man

a) eine Ketocarbonsäure der Formel II

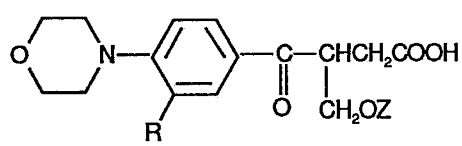

(II)

oder ein reaktionsfähiges Derivat einer solchen Ketocarbonsäure, worin R und Z die oben angegebene Bedeutung haben, mit Hydrazin umsetzt, oder

b) eine Verbindung der Formel III

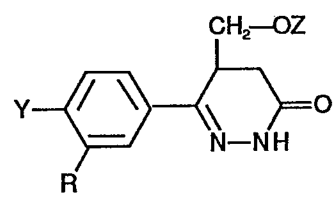

(III)

worin R und Z die unter der Formel I angegebenen Bedeutungen haben, und Y eine zusammen mit Wasserstoff abspaltbare Gruppe bedeutet, mit Morpholin umsetzt, oder

c) eine Verbindung der Formel IV

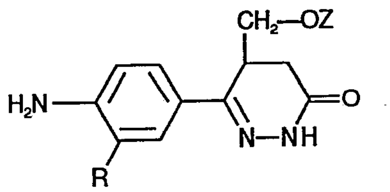

(IV)

mit einem Diäthylätherderivat der Formel V

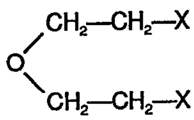

(V)

worin X eine zusammen mit Wasserstoff abspaltbare Gruppe bedeutet und R und Z die oben angegebenen Bedeutungen haben, umsetzt, oder

d) in einer Verbindung der Formel VI

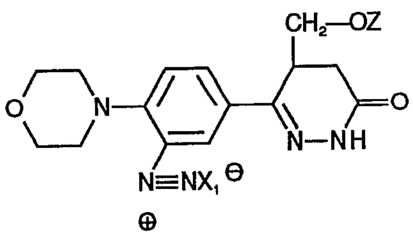

(VI)

worin $X_1^{\ominus}$ ein Anion einer Mineralsäure bedeutet, in Gegenwart von Kupfer oder eines Kupfer-I-salzes ein Halogenatom oder die Cyanogruppe als R einführt, oder

e) eine Verbindung der Formel VII

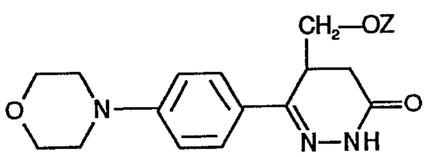

(VII)

halogeniert, oder

f) eine Verbindung der Formel VIII

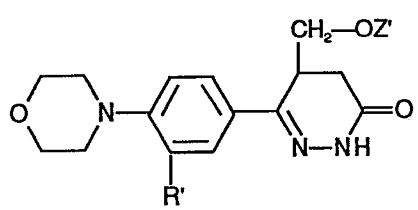

(VIII)

in der R′ die Bedeutung von R hat oder eine leicht solvolysierbare oder hydrogenolysierbare Äthergruppe oder Acyloxygruppe bedeutet und -OZ′ eine leicht solvolysierbare oder hydrogenolysierbare Äther- oder Acyloxygruppe bedeutet, solvolysiert oder hydrogenolysiert und/oder, wenn erwünscht, eine erhaltene Verbindung der Formel I, in der R eine Carboxylgruppe bedeutet in ein Salz umwandelt, und/oder ein erhaltenes Salz der Verbindung der Formel I in eine Verbindung der Formel I, in der R eine freie Carboxygruppe bedeutet, überführt.

**Patentansprüche für den Vertragsstaat AT**

1. Verfahren zur Herstellung von pyridazinonen der Formel I

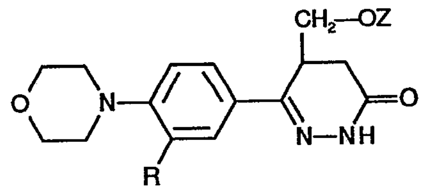

(I)

worin R ein Halogenatom, eine Niederalkyl-, Niederalkoxy-, die Nitro-, Hydroxy-, Cyano-, Carboxy-, Niederalkoxycarbonyl-, Carbamoyl- oder die Trifluormethylgruppe bedeutet, und Z Wasserstoff oder eine substituierte oder unsubstituierte Niederalkanoyl- oder eine Benzoyl- oder eine ein- oder mehrfach durch Halogen, Niederalkyl, Niederalkoxy, Hydroxy, Nitro oder Trifluormethyl substituierte Benzoylgruppe bedeutet, ihre Salze und ihre tautomeren Formen, wobei die mit «nieder» bezeichneten Reste höchstens 7 Kohlenstoffatome aufweisen, dadurch gekennzeichnet, dass man

a) eine Ketocarbonsäure der Formel II

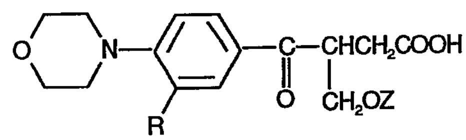

(II)

oder ein reaktionsfähiges Derivat einer solchen Ketocarbonsäure, worin R und Z die oben angegebene Bedeutung haben, mit Hydrazin umsetzt, oder

b) eine Verbindung der Formel III

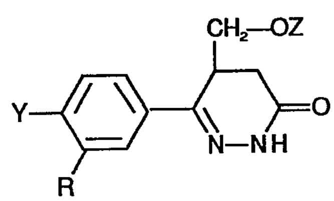

(III)

worin R und Z die unter der Formel I angegebenen Bedeutungen haben, und Y eine zusammen mit Wasserstoff abspaltbare Gruppe bedeutet, mit Morpholin umsetzt, oder

c) eine Verbindung der Formel IV

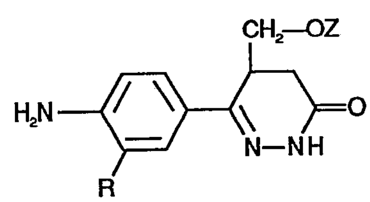

(IV)

mit einem Diäthylätherderivat der Formel V

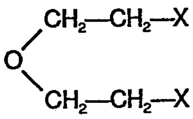

(V)

worin X eine zusammen mit Wasserstoff abspaltbare Gruppe bedeutet und R und Z die oben angegebenen Bedeutungen haben, umsetzt, oder

d) in einer Verbindung der Formel VI

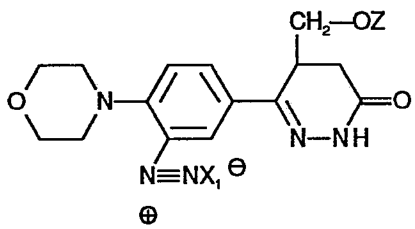

(VI)

worin $X_1^\ominus$ ein Anion einer Mineralsäure bedeutet, in Gegenwart von Kupfer oder eines Kupfer-I-salzes ein Halogenatom oder die Cyanogruppe als R einführt, oder

e) eine Verbindung der Formel VII

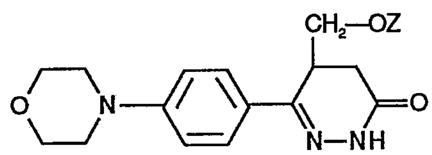

(VII)

halogeniert, oder

f) eine Verbindung der Formel VIII

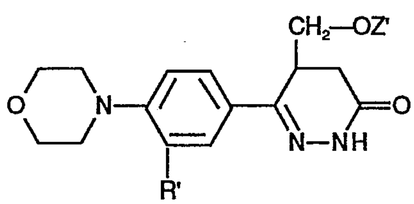

(VIII)

in der R′ die Bedeutung von R hat oder eine leicht solvolysierbare oder hydrogenolysierbare Äthergruppe oder Acyloxygruppe bedeutet und -OZ′ eine leicht solvolysierbare oder hydrogenolysierbare Äther- oder Acyloxygruppe bedeutet, solvolysiert oder hydrogenolysiert und/oder, wenn erwünscht, eine erhaltene Verbindung der Formel I, in der R eine Carboxylgruppe bedeutet in ein Salz umwandelt, und/oder ein erhaltenes Salz der Verbindung der Formel I in eine Verbindung der Formel I, in der R eine freie Carboxygruppe bedeutet, überführt.

2. Verfahren nach Anspruch 1, dadurch gekennzeichnet, dass man Verbindungen der im Anspruch 1 angegebenen allgemeinen Formel I, worin R ein Halogenatom, die Cyano-, Hydroxy-, Carboxy-, Niederalkyl-, Niederalkoxy- oder auch die Trifluormethylgruppe bedeutet und Z die Bedeutung von Wasserstoff oder auch Niederalkanoyl hat, ihre Salze und ihre tautomeren Formen, wobei die mit «nieder» bezeichneten Reste höchstens 7 Kohlenstoffatome aufweisen, herstellt.

3. Verfahren nach Anspruch 1, dadurch gekennzeichnet, dass man Verbindungen der im Anspruch 1 angegebenen allgemeinen Formel I, worin R ein Halogenatom, insbesondere Chlor, eine Niederalkylgruppe, insbesondere Methyl oder auch die Cyanogruppe bedeutet und Z die Bedeutung von Wasserstoff oder auch Niederalkanoyl hat, insbesondere Acetyl, und ihre tautomeren Formen, wobei die mit «nieder» bezeichneten Reste höchstens 7 Kohlenstoffatome aufweisen, herstellt.

4. Verfahren nach Anspruch 1, dadurch gekennzeichnet, dass man 6-(3-Cyano-4-morpholino-phenyl)-5-hydroxymethyl-4,5-dihydro-3(2H)-pyridazinon und seine tautomeren Formen herstellt.

5. Verfahren nach Anspruch 1, dadurch gekennzeichnet, dass man 6-(3-Cyano-4-morpholino-phenyl)-5-acetoxymethyl-4,5-dihydro-3(2H)-pyridazinon und seine tautomeren Formen herstellt.

6. Verfahren nach Anspruch 1, dadurch gekennzeichnet, dass man 6-(3-Chlor-4-morpholino-phenyl)-5-hydroxymethyl-4,5-dihydro-3(2H)-pyridazinon und seine tautomeren Formen herstellt.

7. Verfahren nach Anspruch 1, dadurch gekennzeichnet, dass man 6-(3-Nitro-4-morpholino-phenyl)-5-hydroxymethyl-4,5-dihydro-3(2H)-pyridazinon und seine tautomeren Formen herstellt.

**Claims for the Contracting States: DE GB FR CH LI IT NL BE SE LU**

1. Pyridazinones of the general formula I

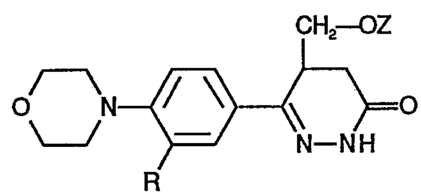

(I)

wherein R is a halogen atom, a lower alkyl or lower alkoxy group, a nitro, hydroxy, cyano, carboxy, lower alkoxy-carbonyl or carbamoyl group or a trifluoromethyl group, and Z is hydrogen or a substituted or unsubstituted lower alkanoyl group, a benzoyl group or a benzoyl group mono- or poly-substituted by halogen, lower alkyl, lower alkoxy, hydroxy, nitro or by trifluoromethyl, and the salts and tautomeric forms thereof, radicals designated "lower" having a maximum of 7 carbon atoms.

2. Pyridazinones of the general formula I according to claim 1, wherein R is a halogen atom, a cyano, hydroxy, carboxy, lower alkyl or lower alkoxy group or a trifluoromethyl group, and Z is hydrogen or lower alkanoyl, and the salts and tautomeric forms thereof, radicals designated "lower" having a maximum of 7 carbon atoms.

3. Pyridazinones of the general formula I according to claim 1, wherein R is a halogen atom, especially chlorine, a lower alkyl group, especially methyl, or a cyano group, and Z is hydrogen or lower alkanoyl, especially acetyl, and the tautomeric forms thereof, radicals designated "lower" having a maximum of 7 carbon atoms.

4. 6-(3-cyano-4-morpholino-phenyl)-5-hydroxymethyl-4,5-dihydro-3(2H)-pyridazinone, and the tautomeric forms thereof.

5. 6-(3-chloro-4-morpholino-phenyl)-5-hydroxymethyl-4,5-dihydro-3(2H)-pyridazinone, and the tautomeric forms thereof.

6. 6-(3-nitro-4-morpholino-phenyl)-5-hydroxymethyl-4,5-dihydro-3(2H)-pyridazinone, and the tautomeric forms thereof.

7. 6-(3-cyano-4-morpholino-phenyl)-5-acetoxymethyl-4,5-dihydro-3(2H)-pyridazinone, and the tautomeric forms thereof,

8. Pharmaceutical preparations containing a compound of the general formula I according to claim 1 and customary carriers.

9. The compounds of the general formula I according to claim 1 for use in a method for the therapeutic treatment of the human or animal body.

10. The compounds of the general formula I according to claim 1 for use as antithrombotically active agents.

11. The use of compounds of the general formula I according to claim 1 and customary carriers for producing pharmaceutical preparations.

12. A process for producing compounds of the general formula I

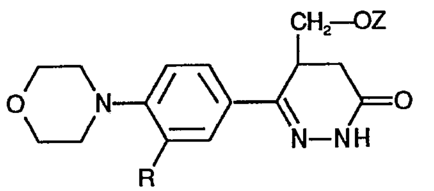

(I)

wherein R is a halogen atom, a lower alkyl or lower alkoxy group, a nitro, hydroxy, cyano, carboxy, lower alkoxycarbonyl or carbamoyl group or a trifluoromethyl group, and Z is hydrogen or a substituted or unsubstituted lower alkanoyl group, a benzoyl group or a benzoyl group mono- or poly-substituted by halogen, lower alkyl, lower alkoxy, hydroxy, nitro or by trifluoromethyl, and the salts and tautomeric forms thereof, radicals designated "lower" having a maximum of 7 carbon atoms, which process comprises:

a) reacting a ketocarboxylic acid of the formula II

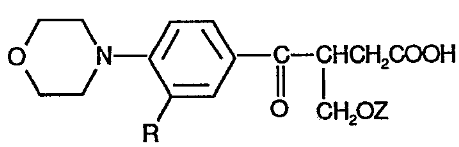

(II)

or a reactive derivative of such a ketocarboxylic acid, wherein R and Z have the meanings given in the foregoing, with hydrazine; or

b) reacting a compound of the formula III

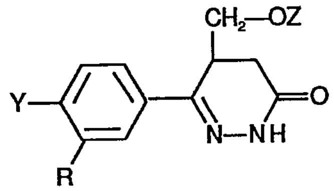

(III)

wherein R and Z have the meanings given under formula I and Y is a group detachable together with hydrogen, with morpholine; or

c) reacting a compound of the formula IV

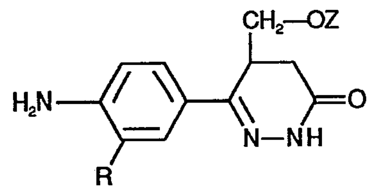

(IV)

with a diethyl ether derivative of the formula V

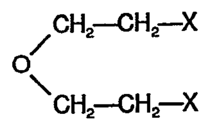

(V)

wherein X is a group detachable together with hydrogen, and R and Z have the meanings given in the foregoing; or

d) introducing a halogen atom or a cyano group as R into a compound of the formula VI

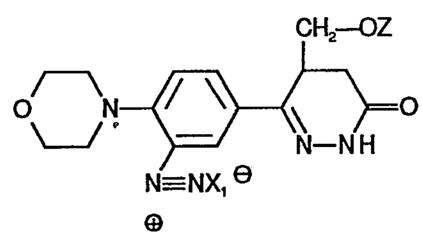

(VI)

wherein $X_1^{\ominus}$ is an anion of a mineral acid, in the presence of copper or of a copper (I) salt; or

e) halogenating a compound of the formula VII

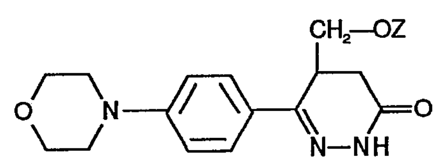

(VII)

or

f) solvolysing or hydrogenolysing a compound of the formula VIII

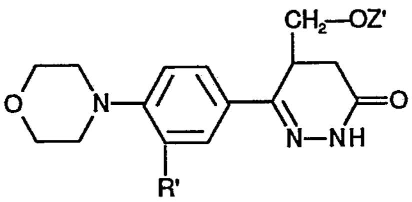

(VIII)

wherein R′ has the meaning of R or is a readily solvolysable or hydrogenolysable ether group or acyloxy group and -OZ′ is a readily solvolysable or hydrogenolysable ether group or acyloxy group;

and/or, if desired, converting a resulting compound of the formula I in which R is a carboxy group into a salt, and/or converting a resulting salt of a compound of the formula I into a compound of the formula I in which R is a free carboxy group.

**Claims for the Contracting State AT**

1. A process for producing pyridazinones of the formula I

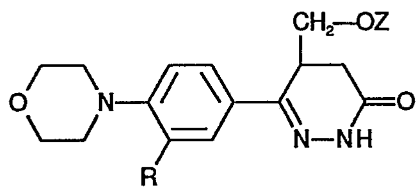

(I)

wherein R is a halogen atom, a lower alkyl or lower alkoxy group, a nitro, hydroxy, cyano, carboxy, lower alkoxycarbonyl or carbamoyl group or a trifluoromethyl group, and Z is hydrogen or a substituted or unsubstituted lower alkanoyl group, a benzoyl group or a benzoyl group mono- or poly-substituted by halogen, lower alkyl, lower alkoxy, hydroxy, nitro or by trifluoromethyl, and the salts and tautomeric forms thereof, radicals designated "lower" having a maximum of 7 carbon atoms, which process comprises:

a) reacting a ketocarboxylic acid of the formula II

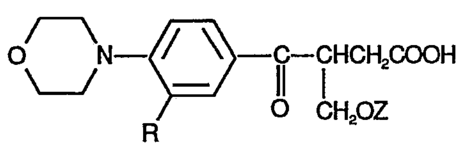

(II)

or a reactive derivative of such a ketocarboxylic acid, wherein R and Z have the meanings given in the foregoing, with hydrazine; or

b) reacting a compound of the formula III

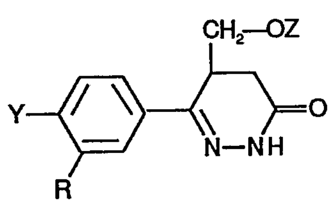

(III)

wherein R and Z have the meanings given under formula I and Y is a group detachable together with hydrogen, with morpholine; or

c) reacting a compound of the formula IV

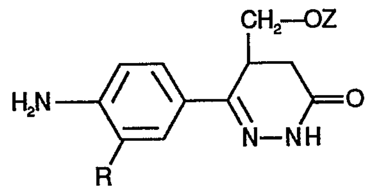

(IV)

with a diethyl ether derivative of the formula V

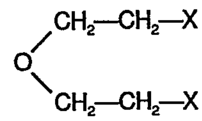

(V)

wherein X is a group detachable together with hydrogen, and R and Z have the meanings given in the foregoing; or

d) introducing a halogen atom or a cyano group as R into a compound of the formula VI

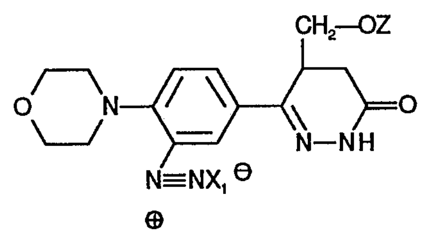

(VI)

wherein $X_1^{\ominus}$ is an anion of a mineral acid, in the presence of copper or of a copper (I) salt; or

e) halogenating a compound of the formula VII

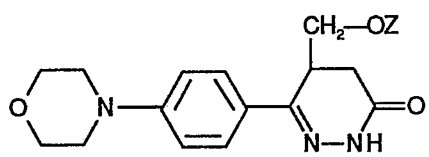

(VII)

or

f) solvolysing or hydrogenolysing a compound of the formula VIII

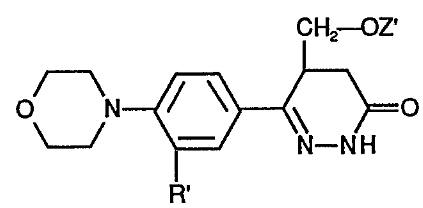

(VIII)

wherein R′ has the meaning of R or is a readily solvolysable or hydrogenolysable ether group or acyloxy group and -OZ′ is a readily solvolysable or hydrogenolysable ether group or acyloxy group;

and/or, if desired, converting a resulting compound of the formula I in which R is a carboxy group into a salt, and/or converting a resulting

salt of a compound of the formula I into a compound of the formula I in which R is a free carboxy group.

2. A process according to claim 1 wherein there are produced compounds of the general formula I given in claim 1 in which R is a halogen atom, or a cyano, hydroxy, carboxy, lower alkyl or lower alkoxy group or a trifluoromethyl group, and Z is hydrogen or lower alkanoyl, and the salts and tautomeric forms thereof, radicals designated "lower" having a maximum of 7 carbon atoms.

3. A process according to claim 1 wherein there are produced compounds of the general formula I given in claim 1 in which R is a halogen atom, especially chlorine, a lower alkyl group, especially methyl, or a cyano group, and Z is hydrogen or lower alkanoyl, especially acetyl, and the tautomeric forms thereof, radicals designated "lower" having a maximum of 7 carbon atoms.

4. A process according to claim 1 wherein there are produced 6-(3-cyano-4-morpholino-phenyl)-5-hydroxymethyl-4,5-dihydro-3(2H)-pyridazinone and the tautomeric forms thereof.

5. A process according to claim 1 wherein there are produced 6-(3-cyano-4-morpholino-phenyl)-5-acetoxymethyl-4,5-dihydro-3(2H)-pyridazinone and the tautomeric forms thereof.

6. A process according to claim 1 wherein there are produced 6-(3-chloro-4-morpholino-phenyl)-5-hydroxymethyl-4,5-dihydro-3(2H)-pyridazinone and the tautomeric forms thereof.

7. A process according to claim 1 wherein there are produced 6-(3-nitro-4-morpholino-phenyl)-5-hydroxymethyl-4,5-dihydro-3(2H)-pyridazinone and the tautomeric forms thereof.

**Revendications pour les Etats contractants: DE GB FR CH LI IT NL BE SE LU**

1. Pyridazinones de formule générale I

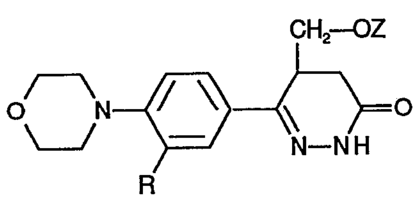

(I)

dans laquelle R représente un atome d'halogène, un groupe alkyle inférieur, alcoxy inférieur, nitro, hydroxy, cyano, carboxy, (alcoxy inférieur)-carbonyle, carbamoyle ou trifluorométhyle et Z représente l'hydrogène ou un groupe alcanoyle inférieure substitué ou non ou un groupe benzoyle ou un groupe benzoyle mono- ou poly-substitué par des halogènes, des groupes alkyle inférieurs, alcoxy inférieurs, hydroxy, nitro ou trifluorométhyle, leurs sels et leurs formes tautomères, les groupes qualifiés d'«inférieurs» contenant au maximum 7 atomes de carbone.

2. Pyridazinones de formule générale I selon la revendication 1, dans laquelle R représente un atome d'halogène, un groupe cyano, hydroxy, carboxy, alkyle inférieur, alcoxy inférieur ou trifluorométhyle et Z représente l'hydrogène ou un groupe alcanoyle inférieur, leurs sels et leurs formes tautomères, l'expression «inférieur» s'appliquant à des groupes qui contiennent au maximum 7 atomes de carbone.

3. Pyridazinones de formule générale I selon la revendication 1, dans laquelle R représente un atome d'halogène, en particulier de chlore, un groupe alkyle inférieur, en particulier méthyle ou encore le groupe cyano et Z représente l'hydrogène ou un groupe alcanoyle inférieur, en particulier un groupe acétyle, et leurs formes tautomères, les groupes qualifiés d'«inférieurs» contenant au maximum 7 atomes de carbone.

4. La 6-(3-cyano-4-morpholino-phényl)-5-hydroxyméthyl-4,5-dihydro-3(2H)-pyridazinone et ses formes tautomères.

5. La 6-(3-chloro-4-morpholino-phényl)-5-hydroxyméthyl-4,5-dihydro-3(2H)-pyridazinone et ses formes tautomères.

6. La 6-(3-nitro-4-morpholino-phényl)-5-hydroxyméthyl-4,5-dihydro-3(2H)-pyridazinone et ses fromes tautomères.

7. La 6-(3-cyano-4-morpholino-phényl)-5-acétoxyméthyl-4,5-dihydro-3(2H)-pyridazinone et ses formes tautomères.

8. Compositions pharmaceutiques contenant l'un des composés de formule générale I selon la revendication 1 et des véhicules usuels.

9. Les composés de formule générale I selon la revendication 1, pour l'utilisation dans un procédé pour le traitement thérapeutique de l'organisme humain ou animal.

10. Les composés de formule générale I selon la revendication 1, pour l'utilisation en tant qu'agents antithrombotiques actifs.

11. L'utilisation des composés de formule générale I selon la revendication 1 et de véhicules usuels pour la préparation de compositions pharmaceutiques.

12. Procédé de préparation des composés de formule générale I

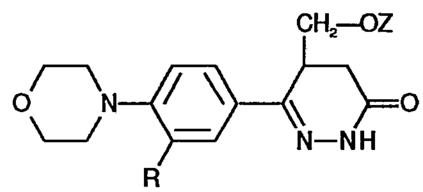

(I)

dans laquelle R représente un atome d'halogène, un groupe alkyle inférieur, alcoxy inférieur, nitro, hydroxy, cyano, carboxy, (alcoxy inférieur)-carbonyle, carbamoyle ou trifluorométhyle et Z représente l'hydrogène ou un groupe alcanoyle inférieur substitué ou non ou un groupe benzoyle ou un groupe benzoyle mono- ou poly-substitué par des halogènes, des groupes alkyle inférieurs,

alcoxy inférieurs, hydroxy, nitro ou trifluorométhyle, de leurs sels et de leurs formes tautomères, les groupes qualifiés d'«inférieurs» contenant au maximum 7 atomes de carbone, caractérisé en ce que:

a) on fait réagir un acide cétocarboxylique de formule II

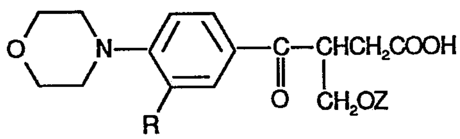
(II)

ou un dérivé réactif d'un tel acide, dans lequel R et Z ont les significations indiquées ci-dessus, avec l'hydrazine, ou bien

b) on fait réagir un composé de formule III

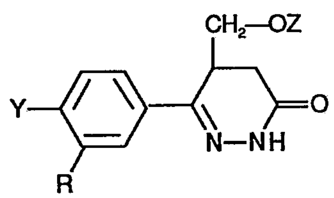
(III)

dans laquelle R et Z ont les significations indiquées ci-dessus et Y représente un groupe éliminable avec l'hydrogène, avec la morpholine, ou bien

c) on fait réagir un composé de formule IV

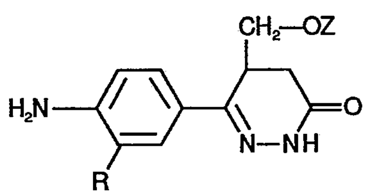
(IV)

avec un dérivé de l'éther diéthylique de formule V

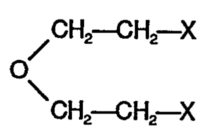
(V)

dans laquelle X représente un groupe éliminable avec l'hydrogène et R et Z ont les significations indiquées ci-dessus, ou bien

d) dans un composé de formule VI

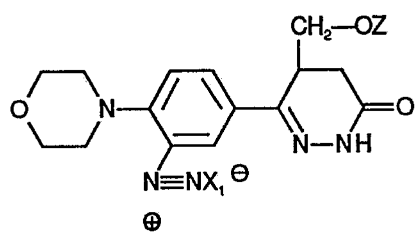
(VI)

dans laquelle $X_1^{\ominus}$ représente un anion d'un acide minéral, on introduit un substituant R consistant en un atome d'halogène ou en le groupe cyano en présence de cuivre ou d'un sel cuivreux, ou bien

e) on halogène un composé de formule VII

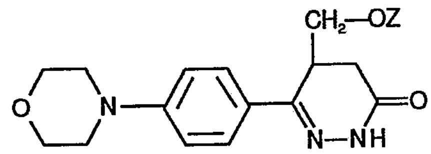
(VII)

ou bien

f) on solvolyse ou on hydrogénolyse un composé de formule VIII

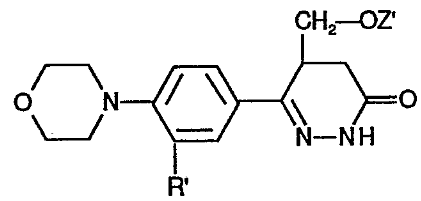
(VIII)

dans laquelle R′ a les mêmes significations que R ou représente un groupe éther ou un groupe acyloxy facile à solvolyser ou à hydrogénolyser et -OZ′ représente un groupe éther ou acyloxy facile à solvolyser ou à hydrogénolyser et, si on le désire, on convertit un composé obtenu répondant à la formule I dans laquelle R représente un groupe carboxyle, en un sel, et/ou on convertit un sel d'un composé de formule I ainsi obtenu en un composé de formule I dans laquelle R représente un groupe carboxy libre.

**Revendications pour l'Etat contractant: AT**

1. Procédé de préparation de pyridazinones de formule I

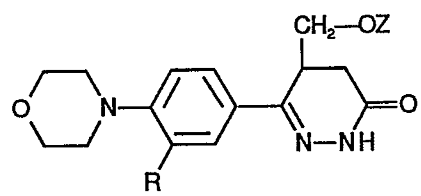
(I)

dans laquelle R représente un atome d'halogène, un groupe alkyle inférieur, alcoxy inférieur, nitro, hydroxy, cyano, carboxy, (alcoxy inférieur)-carbonyle, carbamoyle ou trifluorométhyle et Z représente l'hydrogène ou un groupe alcanoyle inférieur substitué ou non ou un groupe benzoyle ou un groupe benzoyle mono- ou poly-substitué par des halogènes, des groupes alkyle inférieurs, alcoxy inférieurs, hydroxy, nitro ou trifluorométhyle, de leurs sels et de leurs formes tautomères, les groupes qualifiés d'«inférieurs» contenant au maximum 7 atomes de carbone, caractérisé en ce que:

a) on fait réagir un acide cétocarboxylique de formule II

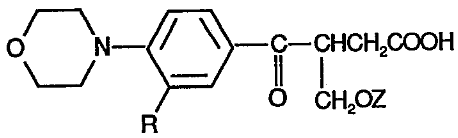

(II)

on un dérivé réactif d'un tel acide, dans lequel R et Z ont les significations indiquées ci-dessus, avec l'hydrazine, ou bien

b) on fait réagir un composé de formule III

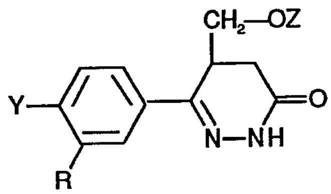

(III)

dans laquelle R et Z ont les significations indiquées ci-dessus et Y représente un groupe éliminable avec l'hydrogène, avec la morpholine, ou bien

c) on fait réagir un composé de formule IV

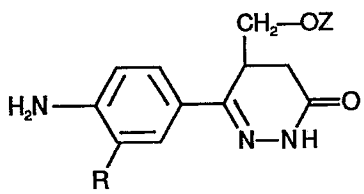

(IV)

avec un dérivé de l'éther diéthylique de formule V

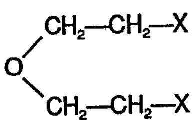

(V)

dans laquelle X représente un groupe éliminable avec l'hydrogène et R et Z ont les significations indiquées ci-dessus, ou bien

d) dans un composé de formule VI

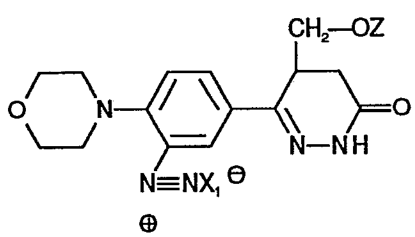

(VI)

dans laquelle $X_1^{\ominus}$ représente un anion d'un acide minéral, on introduit un substituant R consistant en un atome d'halogène ou en le groupe cyano en présence de cuivre ou d'un sel cuivreux, ou bien

e) on halogène un composé de formule VII

ou bien

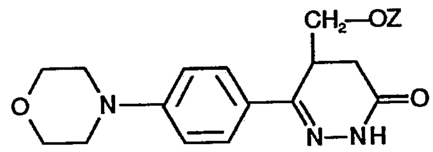

(VII)

f) on solvolyse ou on hydrogénolyse un composé de formule VIII

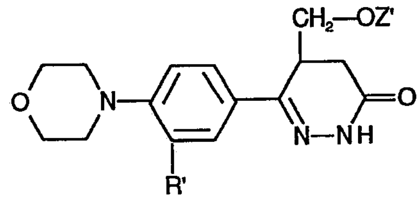

(VIII)

dans laquelle R′ a les mêmes significations que R ou représente un groupe éther ou un groupe acyloxy facile à solvolyser ou à hydrogénolyser et -OZ′ représente un groupe éther ou acyloxy facile à solvolyser ou à hydrogénolyser et, si on le désire, on convertit un composé obtenu répondant à la formule I dans laquelle R représente un groupe carboxyle, en un sel, et/ou on convertit un sel d'un composé de formule I ainsi obtenu en un composé de formule I dans laquelle R représente un groupe carboxy libre.

2. Procédé selon la revendication 1, caractérisé en ce que l'on prépare des composés de formule générale I selon la revendication 1 dans laquelle R représente un atome d'halogène, un groupe cyano, hydroxy, carboxy, alkyle inférieur, alcoxy inférieur ou encore trifluorométhyle et Z représente l'hydrogène ou un groupe alcanoyle inférieur, leurs sels et leurs formes tautomères, les groupes qualifiés d'«inférieurs» contenant au maximum 7 atomes de carbone.

3. Procédé selon la revendication 1, caractérisé en ce que l'on prépare des composés de formule générale I de la revendication 1 dans laquelle R représente un atome d'halogène, en particulier de chlore, un groupe alkyle inférieur, en particulier méthyle, ou encore un groupe cyano et Z représente l'hydrogène ou un groupe alcanoyle inférieur, en particulier un groupe acétyle, et leurs formes tautomères, les groupes qualifiés d'«inférieurs» contenant au maximum 7 atomes de carbone.

4. Procédé selon la revendication 1, caractérisé en ce que l'on prépare la 6-(3-cyano-4-morpholino-phényl)-5-hydroxyméthyl-4,5-dihydro-3(2H)-pyridazinone et ses formes tautomères.

5. Procédé selon la revendication 1, caractérisé en ce que l'on prépare la 6-(3-cyano-4-morpholino-phényl)-5-acétoxyméthyl-4,5-dihydro-3(2H)-pyridazinone et ses formes tautomères.

6. Procédé selon la revendication 1, caractérisé en ce que l'on prépare la 6-(3-chloro-4-morpholino-phényl)-5-hydroxyméthyl-4,5-dihydro-3(2H)-pyridazinone et ses formes tautomères.

7. Procédé selon la revendication 1, caractérisé en ce que l'on prépare la 6-(3-nitro-4-morpholino-phényl)-5-hydroxyméthyl-4,5-dihydro-3(2H)-pyridazinone et ses formes tautomères.